# EUROPÄISCHE PATENTANMELDUNG

(11) **EP 3 260 091 A1**
(43) Veröffentlichungstag der Anmeldung: **27.12.2017**
(21) Anmeldenummer: 17166900.5
(22) Anmeldetag: 18.04.2017
(51) Int. Cl.: A61F 5/01, A61F 5/10

(54) **HANDORTHESE**

(30) Priorität: 24.06.2016 DE 102016111644; 29.08.2016 DE 102016116014
(71) Anmelder: Pattke, Jörg, 12555 Berlin (DE); Lenze, Wolfgang, 32130 Enger (DE)
(72) Erfinder: PATTKE, Jörg, 12555 Berlin (DE)
(74) Vertreter: Wickord, Wiro

(57) **Zusammenfassung**

Handorthese mit einem Handschuh und mindestens einer Fingerschiene, wobei die Fingerschiene durch den Handschuh an einer Hand fixierbar ist und wobei die Fingerschiene mindestens eine Feder aufweist.

## Beschreibung

Die Erfindung betrifft eine Handorthese mit einem Handschuh und mindestens einer Fingerschiene, wobei die Fingerschiene durch den Handschuh an einer Hand fixierbar ist.

Derartige Handorthesen sind aus dem Stand der Technik in zahlreichen Ausführungsvarianten bereits bekannt und dienen dazu, der Progredienz einer Finger- und Daumenverkrümmung entgegenzuwirken oder nach einer operativen Korrektur eine erneute Verkrümmung zu verhindern. Anwendungsgebiete sind vor allem die sogenannte Dupuytren'sche Kontraktur und Fingerverkrümmungen durch Verbrennungsnarben. Bei der Dupuytren'schen Kontraktur bilden sich in der Hohlhand und in den Fingern zwischen Haut und Sehnen bindegewebige Knoten und Stränge, die gutartig sind, aber zu einer zunehmenden Krümmung von Fingern und Daumen führen, einhergehend mit einer zunehmenden Gebrauchsunfähigkeit der betroffenen Hand.

Betroffen von der Dupuytren'schen Erkrankung können alle Finger und der Daumen sein. Die sich ausbildende Verkrümmung kann einen gesamten Finger oder Daumen betreffen. In seltenen Fällen kann es auch zu einer isolierten Kontraktur eines Finger oder Daumengelenks kommen.

Handorthesen der genannten Art werden eingesetzt, um die erkrankten Finger und/oder Daumen, die aktiv nicht mehr vollständig gestreckt werden können, über eine gewisse Zeitspanne in eine Streckhaltung zu drücken oder zu ziehen. Die Handorthesen bewirken also eine passive Streckung und sollen möglichst mehrere Stunden am Stück getragen werden.

Beispielsweise ist aus der DE 20 2011 104 828 U1 eine als Therapiehandschuh ausgebildete Handorthese bekannt. Der Handschuh dient zur Befestigung einer als Fixierschiene ausgebildeten Baugruppe aus Fingerschiene und Grundplatte an einer zu therapierenden Hand, wobei die bekannte Fixierschiene einteilig ausgebildet ist. Die Fixierschiene aus der DE 20 2011 104 828 U1 ist aus Aluminiumblech hergestellt.

Aufgabe der vorliegenden Erfindung ist es, eine Handorthese anzugeben, bei der der Zug oder Druck auf den mit einer Fingerschiene zu streckenden Finger gleichmäßiger verteilt ist und gleichzeitig eine aktive Gegenbewegung des Fingers zulässt.

Zur Lösung der Aufgabe ist die Erfindung in Verbindung mit dem Oberbegriff des Patentanspruchs 1 dadurch gekennzeichnet, dass die Fingerschiene mindestens eine Feder aufweist.

Der Begriff "Finger" umfasst hier sowohl die Langfinger wie auch die Daumen. Entsprechend ist im Folgenden allgemein von "Finger", also beispielsweise "Fingerschiene" die Rede, was nicht einschränkend zu verstehen ist. Sofern geeignet, ist auch eine Verwendung gemeint, die den Daumen betrifft.

Der besondere Vorteil der Erfindung besteht darin, dass eine punktuelle Zug- oder Druckbelastung des gestreckten Fingers oder der gestreckten Finger wirksam vermieden ist. Dies führt zu mehr Tragekomfort und damit zu einer höheren Akzeptanz der erfindungsgemäßen Handorthese bei den Patienten. Die Handorthese ist auf diese Weise länger tragbar, beispielsweise eine ganze Nacht hindurch. Am Tag getragen behindert sie die bestimmungsgemäße Verwendung des zu streckenden Fingers kaum. Insbesondere ist eine aktive Bewegung des Fingers gegen die von der Fingerschiene aufgeprägte Zug- beziehungsweise Druckkraft möglich. Insofern ist auch einer Versteifung des Fingers entgegengewirkt. Die Therapie verläuft insgesamt erfolgreicher. Unerwünschte Nebenwirkungen, wie beispielsweise Schmerzen, Druckgeschwüre oder Durchblutungsstörungen, werden weitestgehend vermieden.

Die erfindungsgemäße Handorthese ist besonders gut geeignet, um von der Dupuytren'schen Kontraktur betroffene Finger mit einer Restkrümmung vom Stadium 1 nach Tubiana, also von etwa 45° oder weniger zu begradigen. Eine derartige Restkrümmung liegt beispielsweise häufig nach einem operativen Eingriff oder einer Nadelfasziotomie vor. Ursache für die Restkrümmung sind insbesondere noch vorhandene Fibrosestränge beziehungsweise die Fibrosestränge umgebendes Narbengewebe, welches typischerweise sehr fest ist. Um dieses feste Gewebe zu dehnen, werden Kräfte im Bereich von 2 N bis 10 N am Endglied beziehungsweise 15 N bis 50 N über das Mittelglied bis zum Grundglied des Fingers benötigt, welche zu Beginn der Behandlung täglich über mehrere Stunden aufgeprägt werden. Bevorzugt erfolgt die Aufprägung einer Zugkraft, indem die Fingerschiene von oben an den Finger angelegt wird und die Zugkraft im Bereich der Fingerunterseite möglichst weiträumig verteilt wird durch ein Textil, welches das zweite und dritte Fingerglied möglichst straff in der Querrichtung aber elastisch in der Längsrichtung und luftig umgreift. Das Textil ist insofern Teil des Handschuhs der Handorthese. Sofern die erfindungsgemäße Fingerorthese regelmäßig eingesetzt beziehungsweise angewendet wird, kann mithin ein chirurgischer Eingriff insgesamt vermieden werden.

Eine besonders vorteilhafte Weiterbildung der erfindungsgemäßen Lehre sieht vor, dass die Feder als eine Blattfeder, insbesondere als ein Federpaket aus mindestens zwei geschichteten Blattfedern, ausgebildet ist. Beispielsweise können die Blattfedern des Federpakets eine gleiche Länge oder eine unterschiedliche Länge aufweisen. Unterschiedliche lange Blattfedern können insbesondere kaskadenartig beziehungsweise gestuft angeordnet sein. Hierdurch ist eine gewünschte Verteilung der durch die Handorthese hervorgerufenen Zug- oder Druckbelastung des betroffenen Fingers auf konstruktiv einfache Weise realisierbar.

Eine vorteilhafte Weiterbildung der vorgenannten Ausführungsform sieht vor, dass das Federpaket derart ausgebildet ist, dass der Zug- oder Druck auf den mit der Fingerschiene gestreckten Finger vom proximalen zum distalen Ende des Fingers abnimmt. Auf diese Weise ist auch eine verbesserte Beweglichkeit des betroffenen beziehungsweise zu therapierenden Fingers erreicht.

Grundsätzlich ist die Fingerschiene nach Art, Anordnung, Dimensionierung, Form und Material in weiten Grenzen frei wählbar. Zweckmäßigerweise erstreckt sich die Fingerschiene, insbesondere die Feder, in einer Gebrauchslage der Handorthese von der gesamten Mittelhand, über das Grundgelenk, das Grundglied und das Mittelgelenk bis zumindest zum Mittelglied des zugeordneten Fingers. Insbesondere vorteilhaft ist es, wenn sich die Fingerschiene, insbesondere die Feder, in einer Gebrauchslage der Handorthese bis zum Endglied des der Handorthese zugeordneten Fingers erstreckt. Im Wesentlichen ist damit die gesamte Länge des betroffenen Fingers in die Therapie einbezogen. Auf diese Weise ist die mögliche Anlagefläche der Fingerschiene an dem betroffenen Finger maximiert. Dies führt unter anderem zu einer geringeren Zug- oder Druckbelastung der einzelnen Fingerbereiche.

Nach einer vorteilhaften Weiterbildung der Erfindung ist die wenigstens eine Blattfeder aus einem elastisch verformbaren, flexiblen Stahl hergestellt. Besonders bevorzugt ist die Verwendung von Federstahl beziehungsweise Bandmaßstahl, wie er bei Rollbandmaßen zum Einsatz kommt. Der Stahl weist beispielsweise eine Dicke im Bereich von 0,1 mm bis 0,3 mm, bevorzugt eine Dicke von 0,15 mm bis 0,25 mm und besonders bevorzugt eine Dicke von 0,16 mm oder 0,2 mm auf. Eine Breite des Stahls entspricht beispielsweise in etwa einer mittleren Breite des zu therapierenden Fingers. Wird der geschiente Finger bewegt und insbesondere gekrümmt, gleitet die Position der Krümmung bei der aus Stahl gefertigten Blattfeder beziehungsweise den Blattfedern sehr gut. Die Feder krümmt sich dabei oberhalb des Fingers, ohne ebendiesen zu berühren. So wird beim aktiven Krümmen des geschienten Fingers eine unvorteilhafte Reibung vermieden und die Belastung des Fingers reduziert. Es ergibt sich insofern ein hoher Tragekomfort.

Nach einer Weiterbildung der Erfindung kann der Federstahl - wie bei Rollbandmaßen üblich - bombiert, das heißt wölbend verformt ausgebildet sein. Die Bombierung beziehungsweise Wölbung ist bevorzugt quer zur Längserstreckung (Längsrichtung) des Federstahls beziehungsweise der Fingerschiene realisiert. Besonders bevorzugt ist die Bombierung des Federstahls über die gesamte Länge desselben gleich ausgeformt, so dass eine einfache Fertigung möglich ist und sich eine hohe Lagetoleranz beim Anlegen der Fingerschiene ergibt.

Die bombiert ausgebildeten Blattfedern werden mit ihrer hohlen Seite auf die Fingeroberseite angelegt. Bei einem Federpaket sind vorzugsweise alle Federn in gleicher Art bombiert ausgebildet. Die Bombierung wird hergestellt beispielsweise durch Walzen, Abkanten, Biegen oder Tiefziehen.

Vorteilhaft erlaubt es die Bombierung, dass bei einer Krümmung des Fingers sich über den Gelenken eine Brücke im Federstahl ausbildet mit der Folge, dass auch bei einer Krümmung eine Anlage des Federstahls am Fingergelenk und damit eine ungünstige Druckbelastung in diesem Bereich vermieden sind. Darüber hinaus ergibt sich eine im Wesentlichen gleiche Federkraft über die verschiedenen Krümmungswinkel des Fingers und die Federkraft fällt im Wesentlichen plötzlich beziehungsweise sofort ab, wenn eine gerade Stellung für die Blattfeder und den daran festgelegten Finger erreicht ist. Zudem verdoppelt sich durch die Bombierung infolge der Versteifung des Materials die Federkraft bezogen auf das verwendete Material, das heißt ein gleicher Materialeinsatz sorgt für eine größere Federkraft. Die Fingerorthese kann insofern sehr schlank und leicht ausgeführt sein.

Alternativ kann mindestens eine Blattfeder des Federpakets aus einem Kunststoff und bevorzugt aus einem faserverstärkten Kunststoff hergestellt sein. Insbesondere faserverstärkte Kunststoffe sind leicht und robust. Die mechanischen Eigenschaften von faserverstärkten Kunststoffen lassen sich darüber hinaus in gewünschter Weise über eine Vielzahl von Parametern einstellen. Beispielsweise lässt sich über die Ausrichtung der Fasern das Elastizitätsverhalten richtungsabhängig gestalten. Das aus mindestens zwei Blattfedern bestehende Federpaket kann auf dem Fachmann bekannte und geeignete Weise gebildet und befestigt sein.

Vorteilhaft ist es, wenn die Fingerschiene an der Außenhand angeordnet und der zugeordnete Finger damit auf Zug beansprucht ist. Hierdurch ist der zu therapierende Finger aber auch die betroffene Hand als Ganzes in deren Funktion weniger beeinträchtigt.

Eine konstruktiv besonders einfache Realisierung der vorgenannten Ausführungsform sieht vor, dass die Feder im Bereich des Grundgliedes des zugeordneten Fingers derart zweifach geknickt ausgebildet ist, dass die Feder einen versteiften beziehungsweise im Wesentlichen starren Mittelbereich aufweist, von dem aus sich ein erster federnder Endbereich in Richtung der Mittelhand und ein zweiter federnder Endbereich in Richtung des Mittelgliedes des zugeordneten Fingers erstreckt. Ist eine größere Steifheit des Mittelbereiches der Feder erforderlich, so kann an den Mittelbereich der Feder zusätzlich eine Versteifungsplatte angeordnet sein. Beim Vorsehen von einer Mehrzahl von geschichteten Blattfedern können diese in dem Mittelbereich mechanisch verbunden sein. Beispielsweise kann eine Nietverbindung vorgesehen sein zum Verbinden der Blattfedern und zum gleichzeitigen Ausbilden des Mittelbereichs.

Eine dazu alternative Ausführungsform sieht vor, dass die Fingerschiene im Bereich des Grundgliedes des zugeordneten Fingers eine Fingerplatte aufweist, wobei an der Fingerplatte mindestens eine erste und mindestens eine zweite Feder angeordnet sind, von denen sich die erste Feder in Richtung der Mittelhand und die zweite Feder in Richtung des Mittelgliedes des zugeordneten Fingers erstreckt. Somit entspricht die erste Feder dem ersten federnden Endbereich und die zweite Feder dem zweiten federnden Endbereich der vorherigen Ausführungsform. Hierdurch ist die Anpassung der einzelnen Federbereiche auf die Erfordernisse des Einzelfalls besonders einfach möglich. Beispielsweise können die erste und die zweite Feder nach Art, Material, Anzahl und Form voneinander verschieden ausgebildet sein. Die Geometrie der Fingerplatte kann an die Geometrie der Hand beziehungsweise den Grad der Fingerkrümmung angepasst verformt sein.

Die Fingerplatte kann auch Teil eines Mittelmoduls sein. Das Mittelmodul wird dann mit der Fingerplatte im Bereich des Grundglieds des zugeordneten Fingers angelegt. Zugleich kann das Mittelmodul ausgebildet sein zur Aufnahme einer variablen und veränderbaren Anzahl von Federn, welche sich in Richtung der Mittelhand einerseits beziehungsweise des Mittelglieds des zugeordneten Fingers andererseits erstrecken. Beispielsweise können an dem Mittelmodul Aufnahmestifte für die Federn vorgesehen sein und die Federn durch eine Andrückplatte, welche lösbar in Bezug zur Fingerplatte gehalten ist, festgelegt werden. Zur lösbaren Festlegung kann beispielsweise eine Verschraubung vorgesehen sein. Vorteilhaft kann durch das Mittelmodul die Federkraft durch die Wahl einer geeigneten Anzahl von Federn und/oder eine Anpassung der Federgeometrie an dem Behandlungsverlauf angepasst werden. Es kann beispielsweise durch die Reduzierung der Federn im Verlaufe der Behandlung die Federkraft reduziert oder die Verteilung der Federkraft in die Fingerlängsrichtung durch die Änderung der Länge, Breite und Dicke der einzelnen Federn verändert werden.

Sofern eine isolierte Kontraktur über dem Endglied des Fingers ausgebildet ist, kann die Fingerplatte beziehungsweise der starre Mittelbereich dem Mittelglied des Fingers statt dem Grundlied zugeordnet beziehungsweise an das Mittelglied angelegt sein.

Aus den weiteren Unteransprüchen und der nachfolgenden Beschreibung sind weitere Vorteile, Merkmale und Einzelheiten der Erfindung zu entnehmen. Dort erwähnte Merkmale können jeweils einzeln für sich oder auch in beliebiger Kombination erfindungswesentlich sein. So kann auf die Offenbarung zu den einzelnen Erfindungsaspekten stets wechselseitig Bezug genommen werden. Die Zeichnungen dienen lediglich beispielhaft der Klarstellung der Erfindung und haben keinen einschränkenden Charakter.

Es zeigen:
- Fig. 1A: ein Ausführungsbeispiel einer Fingerschiene einer erfindungsgemäßen Handorthese in teilweiser Darstellung und in einer Seitenansicht;
- Fig. 1B: das Ausführungsbeispiel der Fingerschiene aus Fig. 1A in einer teilweisen Draufsicht;
- Fig. 2: das Ausführungsbeispiel der Fingerschiene aus Fig. 1 in einer ersten Gebrauchslage in perspektivischer, teilweiser Darstellung;
- Fig. 3: das Ausführungsbeispiel der Fingerschiene aus Fig. 1 in einer zweiten Gebrauchslage in einer weiteren perspektivischen, teilweisen Darstellung,
- Fig. 4: das Ausführungsbeispiel aus Fig. 1 in einer dritten Gebrauchslage in einer perspektivischen Darstellung mit Handschuh,
- Fig. 5: eine perspektivische Darstellung eines zweiten Ausführungsbeispiels der erfindungsgemäßen Fingerschiene mit bombiert ausgebildeten Blattfedern,
- Fig. 6: eine erste Querschnittsgeometrie einer Blattfeder der Fingerschiene nach Fig. 5,
- Fig. 7: eine alternative zweite Querschnittsgeometrie einer Blattfeder der Fingerschiene nach Fig. 5,
- Fig. 8: eine alternative dritte Querschnittsgeometrie einer Blattfeder der Fingerschiene nach Fig. 5,
- Fig. 9: eine alternative vierte Querschnittsgeometrie einer Blattfeder der Fingerschiene nach Fig. 5 und
- Fig. 10: das Ausführungsbeispiel der erfindungsgemäßen Fingerschiene nach Fig. 5 in einer perspektivischen Darstellung mit Handschuh.

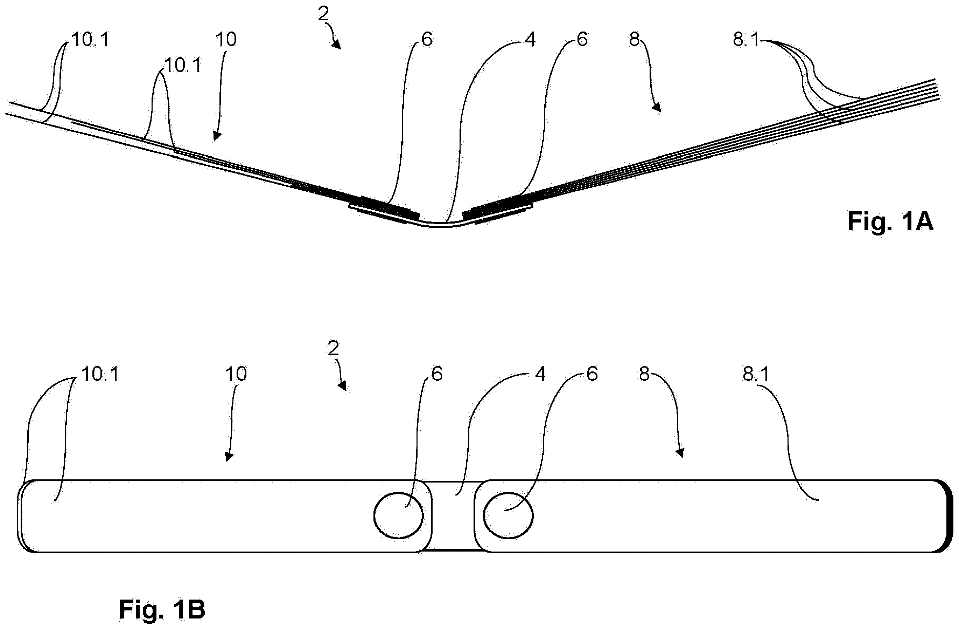

In Fig. 1A ist ein Ausführungsbeispiel einer erfindungsgemäßen Handorthese mit einer Fingerschiene 2 teilweise dargestellt. Die Fingerschiene 2 weist hier eine Fingerplatte 4 aus Aluminium auf, an der über Nietverbindungen 6 eine erste Feder 8 und eine zweite Feder 10 befestigt sind. Die erste Feder 8 und die zweite Feder 10 sind jeweils als Federpakete 8, 10 aus mehreren geschichteten Blattfedern 8.1, 10.1 aus Bandmaßstahl ausgebildet. Grundsätzlich können die Blattfedern 8.1, 10.1 jedoch auch aus einem anderen flexiblen, elastisch verformbaren Stahl, aus Kunststoff und insbesondere aus einem faserverstärkten Kunststoff oder dergleichen ausgebildet sein.

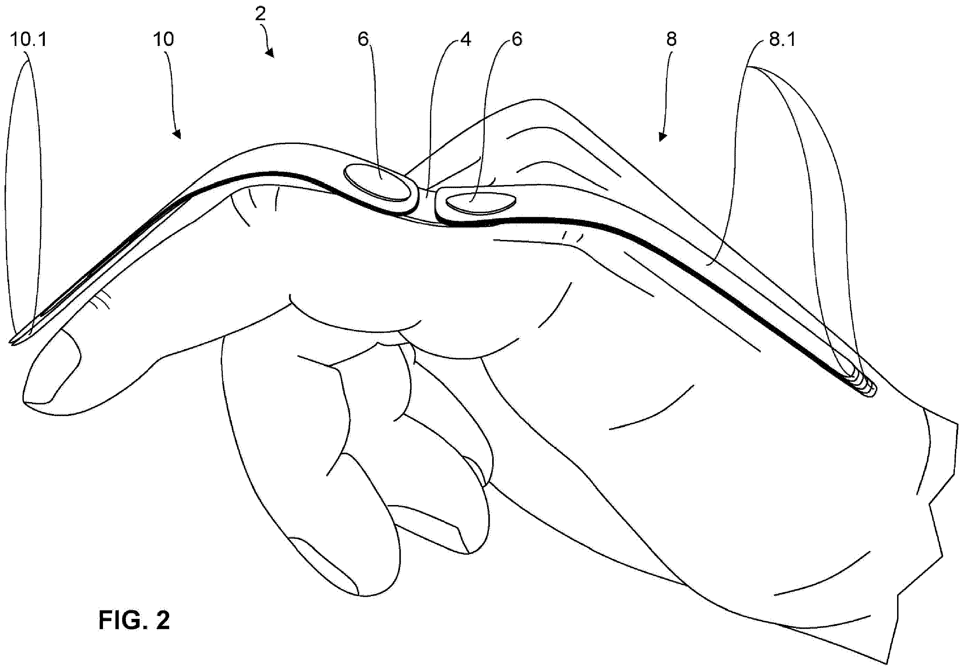

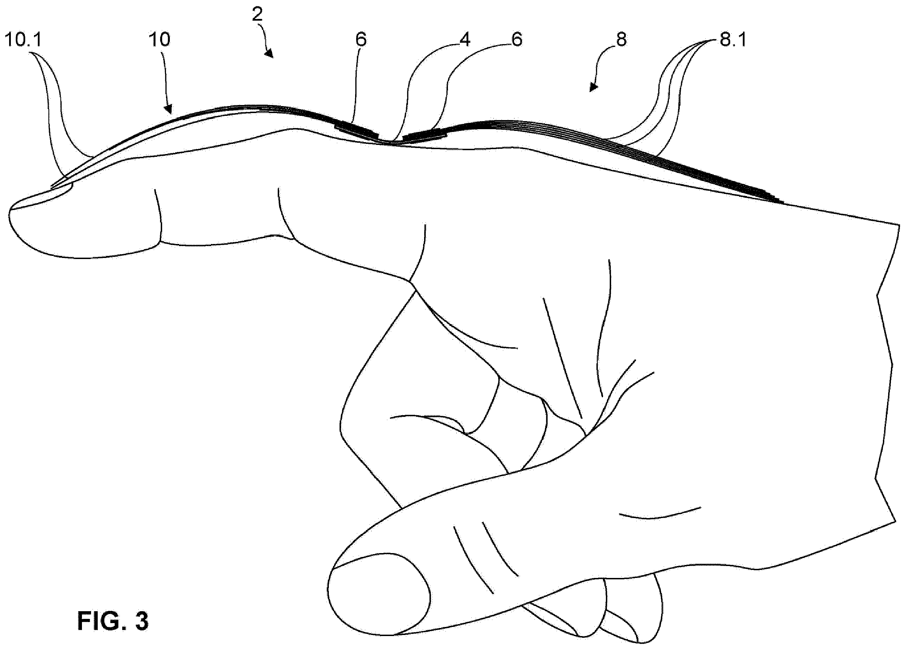

Das erste Federpaket 8 erstreckt sich mit dessen freien Enden, also dessen federnden Endbereichen, in Richtung der Mittelhand und das zweite Federpaket 10 erstreckt sich mit dessen freien Enden, also dessen federnden Endbereichen, in Richtung des Mittelglieds beziehungsweise Endglieds des betroffenen Fingers, also des der Handorthese zugeordneten Fingers. Die Mittelhand und der mit der Handorthese zu therapierende Finger sind lediglich in den Fig. 2 und 3 dargestellt.

Das erste Federpaket 8 weist hier sechs gleichlange Blattfedern 8.1 auf, während das zweite Federpaket 10 drei innenliegende Blattfedern 10.1 und zwei außenliegende Blattfedern 10.1 aufweist. Die innenliegenden Blattfedern 10.1 sind zueinander verschieden lang ausgebildet, beginnend mit der kürzesten Blattfeder 10.1 auf der der Fingerplatte 4 zugewandten Seite des zweiten Federpakets 10. Die beiden außenliegenden Blattfedern 10.1 sind zueinander gleich lang ausgebildet und überragen die innenliegenden Blattfedern 10.1. Auf diese Weise sind die innenliegenden Blattfedern 10.1 durch die außenliegenden Blattfedern 10.1 eingefasst, was eine Fixierung in dem in der Fig. 1A nicht dargestellten Handschuh und damit an der betroffenen Hand und dem zu therapierenden Finger erleichtert.

Die Fingerschiene 2 mit der Fingerplatte 4 und den beiden Federpaketen 8, 10 ist derart ausgebildet, dass der zugeordnete Finger durch die Handorthese in deren Gebrauchslage auf Zug beansprucht ist, wobei der Zug auf den mit der Fingerschiene 2 gestreckten Finger vom proximalen zum distalen Ende des Fingers abnimmt. Siehe hierzu auch die in den Fig. 2 und 3 dargestellten Gebrauchslagen der Handorthese.

Die Fingerschiene 2 ist also auf der Handaußenseite der betroffenen Hand angeordnet. Auf diese Weise wird die Funktion der Hand und der Finger, nicht nur des zu therapierenden Fingers, weniger beeinträchtigt als bei einer Anordnung der Fingerschiene an der Handinnenseite. Insbesondere gilt dies, falls mehrere Finger zu behandeln sind und damit mehrere Fingerschienen gleichzeitig eingesetzt werden.

Fig. 1B zeigt das Ausführungsbeispiel aus Fig. 1A in einer teilweisen Draufsicht, also - bezogen auf die Blattebene - mit Blickrichtung von oben auf Fig. 1A.

Fig. 2 zeigt die Handorthese aus Fig. 1A und Fig. 1B in einer ersten Gebrauchslage an einer betroffenen Hand. Die Handorthese ist in der Fig. 2 ohne den Handschuh dargestellt, um die Übersichtlichkeit zu verbessern. Die Handorthese ist an den betroffenen Finger, hier dem kleinen Finger, angelegt und mittels des Handschuhs nicht nur an der Fingerbeere des kleinen Fingers und an der Mittelhand fixiert. Vielmehr ist die Fingerschiene 2 über den Handschuh großflächig mit dem zu therapierenden Finger in Anlage und damit in Kraftübertragungsverbindung mit dem betroffenen Finger. Der Vorteil hiervon ist, dass die Zugbelastung des kleinen Fingers über im Wesentlichen den gesamten Finger beziehungsweise dem End- und Mittelglied verteilt ist. Entsprechend gering ist die Belastung der einzelnen Fingerbereiche. In der ersten Gebrauchslage ist der kleine Finger gekrümmt dargestellt.

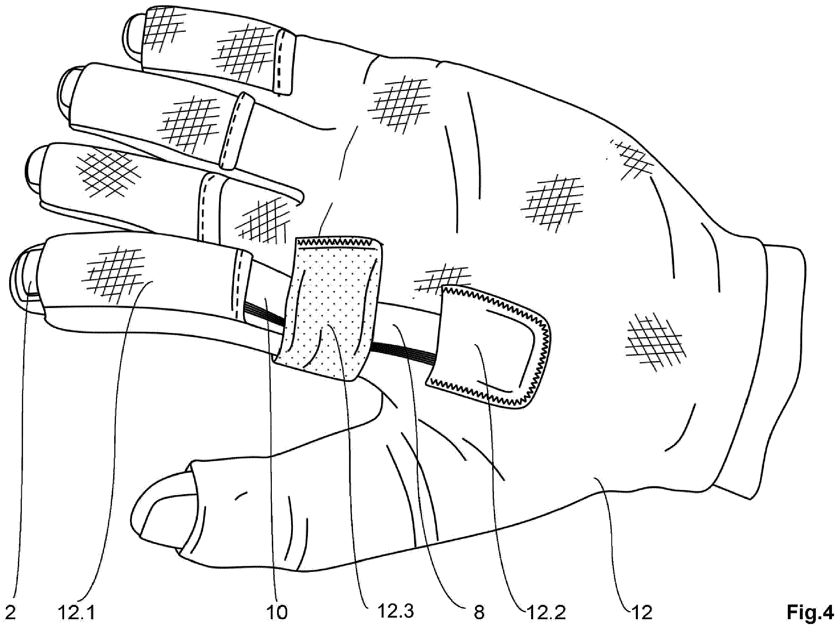

Die Fingerschiene 2 ist an einem lediglich in Fig. 4 dargestellten Handschuh 12 angeordnet und mittels des Handschuhs 12 an der zu therapierenden Hand fixiert und positioniert. Beispielsweise kann die Fingerschiene 2 über Befestigungstaschen 12.1, 12.2 und -lasche 12.3 an dem Handschuh 12 befestigt sein. Die Fingerschiene 2 kann sogar integraler Bestandteil des Handschuhs 12 sein. Der Handschuh 12 weist bei dem vorliegenden Ausführungsbeispiel zur besseren Positionierung der Fingerschiene 2 eine dem zu therapierenden Finger zugeordnete, als Einstecktasche ausgebildete erste Befestigungstasche 12.1 und eine über dem Handrücken vorgesehene zweite Befestigungstasche 12.2 auf. Eine im Bereich der Fingerplatte 4 vorgesehene Befestigungslasche 12.3 ist vorliegend als Klettverschluss ausgebildet. Durch die Befestigungslasche 12.3 lässt sich die Handorthese auf besonders einfache Weise, auch seitens des Patienten, fixieren.

Wie aus Fig. 2 deutlich erkennbar ist, liegt die Fingerschiene 2 in einer gekrümmten Position des Fingers mit der Fingerplatte 4 an dem Grundglied des kleinen Fingers an. Eventuell erfolgt die Anlage nicht unmittelbar, sondern mittelbar, beispielsweise wenn die Fingerschiene 2 wie in dem vorliegenden Ausführungsbeispiel nach Fig. 4 in diesem Bereich in eine Befestigungstasche beziehungsweise -lasche 12.1, 12.2, 12.3 des Handschuhs 12 eingesteckt ist. Das erste Federpaket 8 erstreckt sich, ausgehend von der Fingerplatte 4, in Richtung Mittelhand. Das Maß der Anlage des ersten Federpakets 8 an der Mittelhand bestimmt hierbei die auf den geschienten Finger wirkende Zugkraft mit. Das zweite Federpaket 10 erstreckt sich in der Gebrauchslage der Handorthese ausgehend von der Fingerplatte 4 bis zum Mittelglied beziehungsweise Endglieds des kleinen Fingers. Sofern der Finger gestreckt ist, muss die Fingerplatte 4 nicht an dem Grundglied anliegen. Sie wird bevorzugt von der Feder 8 beabstandet zum Finger gehalten und schwebt quasi über dem Grundglied mit der Folge, dass ein Druck reduziert und der Tragekomfort begünstigt wird.

Der kleine Finger wird durch die Handorthese auf Zug belastet. Hierfür stützen sich die beiden Federpakete 8, 10 an der an dem Grundglied anliegenden Fingerplatte 4 ab. Die Fingerplatte 4 ist entsprechend in Fingerstreckrichtung gebogen; siehe hierzu auch Fig. 1A. Je nach Krümmungsgrad der Fingerplatte 4 lässt sich somit die durch die Handorthese auf den zugeordneten Finger ausgeübte Zugbelastung einstellen beziehungsweise vorbestimmen. Der Biegegrad der Fingerplatte 4 ist also ein weiterer Parameter, um die gewünschte Zugbelastung zu realisieren.

Ebenfalls aus Fig. 2 ist ersichtlich, dass sich die Fingerschiene 2 der Handorthese hier in der ersten Gebrauchslage der Handorthese von der Mittelhand, über das Grundgelenk, das Grundglied, das Mittelgelenk, das Mittelglied und das Endgelenk bis zu der Fingerbeere des kleinen Fingers erstreckt. Ferner ist erkennbar, dass das erste Federpaket 8 hier im Wesentlichen zu einem Drittel im Bereich des Grundgliedes des kleinen Fingers und zu zwei Drittel im Bereich der Mittelhand angeordnet ist.

Fig. 3 zeigt das Ausführungsbeispiel in einer zweiten Gebrauchslage. Im Unterschied zu der Fig. 2 ist hier der Zeigefinger als der zu therapierende Finger dargestellt. Dies dient lediglich dazu, um die flexible Anwendung der ansonsten identischen Grundkonstruktion zu verdeutlichen. In Fig. 3 ist der zu therapierende Finger gestreckt dargestellt; vergleiche Fig. 2. Auch hier ist der Übersichtlichkeit wegen die Handorthese ohne den Handschuh 12 dargestellt.

In Fig. 4 ist die vollständige Handorthese des vorliegenden Ausführungsbeispiels in einer Gebrauchslage analog zur Fig. 3 dargestellt. Wie bereits zur Fig. 3 ausgeführt, ist es für die Erläuterung des Ausführungsbeispiels unerheblich, ob es sich bei dem zu therapierenden Finger um den kleinen Finger gemäß Fig. 2 oder um den Zeigefinger gemäß der Fig. 3 und 4 handelt. Der grundsätzliche Aufbau der Handorthese gemäß dem vorliegenden Ausführungsbeispiel ist identisch.

Deutlich zu erkennen ist die Befestigungstasche 12.1 im Bereich des Mittelgliedes und des Endgliedes des zu therapierenden Fingers, in die das Federpaket 10 eingesteckt ist, die als Klettverschluss ausgebildete Befestigungslasche 12.3 im Bereich des Grundgliedes des Zeigefingers, durch die die in Fig. 4 nicht dargestellte Fingerplatte 4 auf einfache Weise verstellbar fixiert ist und die als Einstecktasche ausgebildete Befestigungstasche 12.2, in die das Federpaket 8 mit dessen freiem Ende eingesteckt ist.

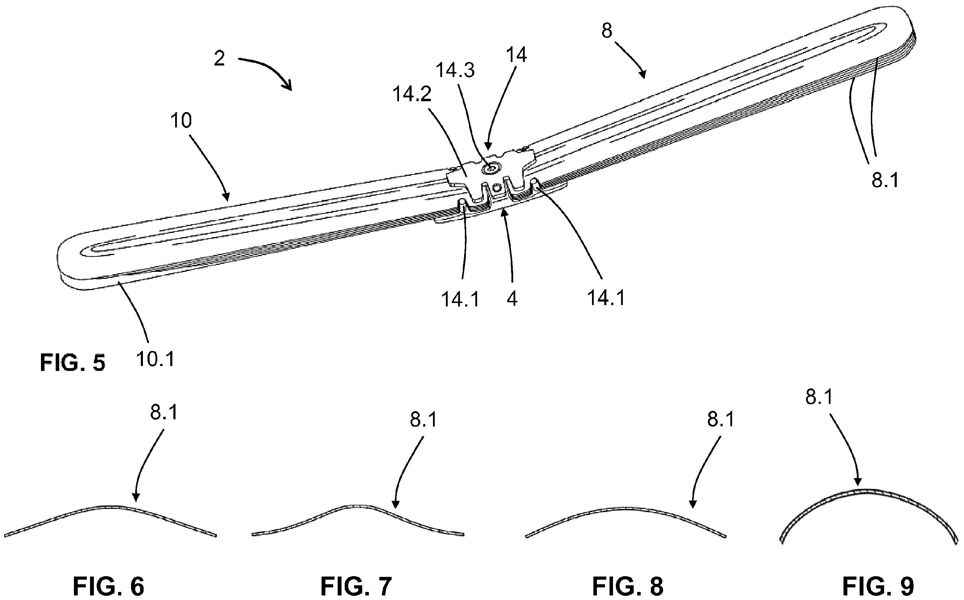

Eine alternative Ausführungsform der Fingerschiene 2 nach Fig. 5 sieht wie gehabt ein erstes Federpaket 8 sowie ein zweites Federpaket 10 vor, welche vorliegend durch ein die Fingerplatte 4 aufweisendes Mittelmodul 14 verbunden sind. Die Federpakete 8, 10 sind dabei lösbar an dem Mittelmodul 14 festgelegt. Das Mittelmodul 14 weist insofern eine Mehrzahl von Stiften 14.1 auf, welche der Festlegung der Blattfedern 8.1, 10.1 der Federpakete 8, 10 dienen. An den Blattfedern 8.1, 10.1 sind insofern korrespondierend zu den Stiften 14.1 des Mittelmoduls 14 Ausnehmungen 8.2, 10.2 gebildet. Ferner sieht das Mittelmodul 14 eine Andrückplatte 14.2 vor, welche über eine Schraube 14.3 mit der Fingerplatte 4 lösbar verbunden ist. Zwischen der Fingerplatte 4 und der Andrückplatte 14.2 sind dabei die Blattfedern 8.1, 10.1 der Federpakete 8, 10 festgelegt.

Das Vorsehen des Mittelmoduls 14 mit der lösbar festgelegten Andrückplatte 14.2 erlaubt es, die Anzahl der Blattfedern 8.1, 10.1 sowie die Geometrie derselben variabel zu wählen und insbesondere im Verlaufe der Behandlung dem Behandlungsfortschritt anzupassen. Zugleich gewährleistet die Fingerplatte 4, welche im Bereich des Grundglieds von oben an den Finger angelegt wird, dass der Tragekomfort erhalten beziehungsweise nicht negativ beeinflusst wird.

Exemplarisch ist in der Fig. 5 dargestellt, dass die Blattfedern 8.1, 10.1 bombiert ausgebildet sind. Die gewölbte Verformung ist dabei so realisiert, dass die Fingerschiene 2 mit der hohlen Seite von oben an den geschienten Finger und auf den Handrücken angelegt wird. Infolge der Bombierung kann der Finger gekrümmt werden, wobei auch bei einer Krümmung oberhalb eines Gelenks des Fingers die Federpakete 8, 10 eine Brücke bilden und beabstandet zu dem Gelenk vorgesehen sind. Bevorzugt ist die Brücke immer genau über dem Gelenk mittig gebildet. Es wird durch die Brückenbildung insbesondere ein mechanischer Kontakt vermieden und einer unangenehmen Druckbelastung vorgebeugt. Der Tragekomfort der erfindungsgemäßen Fingerschiene erhöht sich hierdurch.

Alternative Ausführungsformen der Bombierung sind exemplarisch in den Fig. 6 bis 9 dargestellt. Die Fig. 6 bis 9 zeigen eine einzelne Blattfeder 8.1 exemplarisch im Querschnitt, das heißt quer zur Längserstreckung der Blattfeder 8.1. Bevorzugt ist vorgesehen, dass alle Blattfedern 8.1, 10.1 der Federpakete 8, 10 in der gleichen Art bombiert sind und insofern unmittelbar flächig aneinander angelegt werden können.

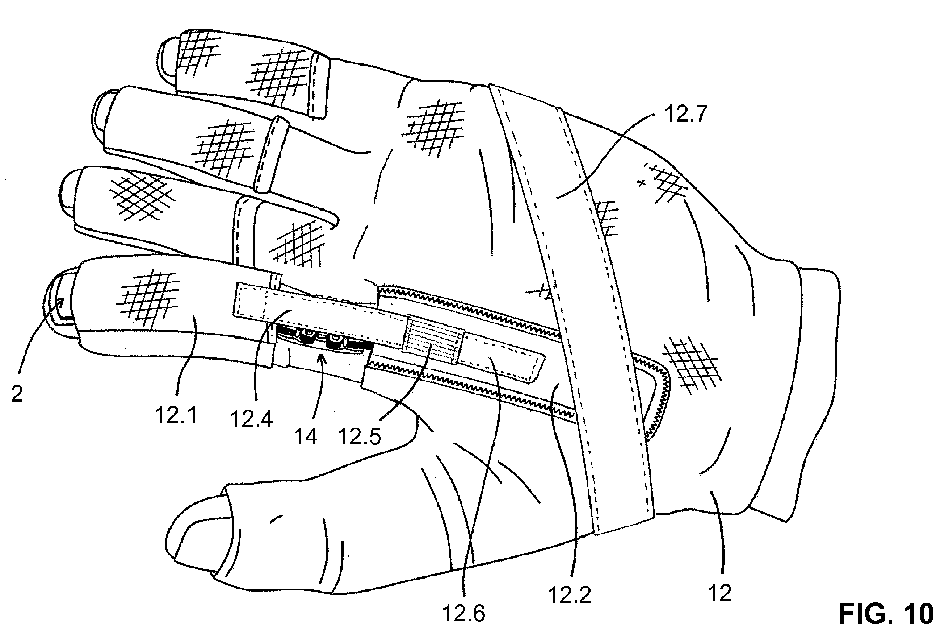

Die Fingerschiene 2 nach Fig. 5 ist in Fig. 10 in einen Handschuh 12 eingesetzt. Es sind dazu das erste Federpaket 8 und das zweite Federpaket 10 in die Befestigungstaschen 12.1, 12.2 des Handschuhs 12 eingelegt. Die Positionierung der Fingerschiene 2 an der Hand erfolgt so, dass das Mittelmodul 14 mit der Fingerplatte 4 über dem Grundglied des geschienten Fingers vorgesehen ist. Die Federpakete 8, 10 mit den bombiert ausgebildeten Federn 8.1, 10.1 werden mit der hohlen Seite von oben an den Finger und auf den Handrücken gelegt.

Die Fixierung der Fingerschiene 2 an dem Handschuh 12 beziehungsweise der Hand erfolgt vorliegend zum einen über ein Halteband 12.4, welches von der das zweite Federpaket 10 aufnehmenden ersten Befestigungstasche 12.1 in Richtung der zweiten Befestigungstasche 12.2 geführt ist, ein sich an das Halteband 12.4 anschließendes flexibles Band 12.5 sowie ein Klettband 12.6, welches mit dem flexiblen Band 12.5 verbunden ist und lösbar auf der zweiten Befestigungstasche 12.2 festgelegt wird. Die Oberfläche der Befestigungstasche 12.2 sowie das Klettband 12.6 bilden insofern zwei Partner einer Klettverbindung.

Weiter erfolgt die Festlegung über einen Spanngurt 12.7, welcher in der Gebrauchslage über den Handrücken geführt ist und das erste Federpaket 8 gegen den Handrücken anlegt. In dem Spanngurt 12.7 ist bevorzugt ein Federelement eingelegt, welches eine variable Kraft auf das erste Federpaket 8 ausübt und zugleich den Tragekomfort erhöht und eine sichere Anlage begünstigt.

Die Erfindung ist nicht auf das anhand der Fig. 1 bis 10 erläuterte Ausführungsbeispiel begrenzt.

Obwohl sich das vorliegende Ausführungsbeispiel auf die Verwendung der erfindungsgemäßen Handorthese bei einer menschlichen Hand bezieht wäre eine Verwendung bei einer tierischen Hand ebenfalls denkbar.

Im Einzelfall kann es zweckmäßig sein, die mindestens eine Fingerschiene der erfindungsgemäßen Handorthese in der Gebrauchslage an der Handinnenseite zu platzieren. Entsprechend würden der oder die erkrankten Finger durch eine derartige Handorthese auf Druck beansprucht. Möglich ist auch, dass sowohl an der Handinnenseite wie auch an der Handaußenseite Fingerschienen angeordnet sind.

Derartige Fingerschienen könnten dann voneinander verschiedenen Fingern oder auch einem einzigen zu therapierenden Finger zugeordnet sein.

Der Fachmann wird je nach Anwendungsfall die erforderliche Anzahl an Blattfedern und deren Längen für jeden einzelnen zu therapierenden Finger auswählen. Entsprechend muss die Anzahl der Blattfedern nicht zwingend bei jedem Federpaket oder bei jeder Fingerschiene identisch sein.

Eine Anstellung der Federpakete 8, 10 zueinander unter einem flachen Winkel kann wie in dem Ausführungsbeispiel nach Fig. 1A erreicht werden, indem die Fingerplatte 4 gekrümmt ausgebildet ist. Beispielsweise kann die Anstellung erreicht werden durch Keileinlagen, welche zwischen den Federpaketen 8, 10 und der Fingerplatte 4 vorgesehen werden. Die Keileinlagen können insofern auch bei dem Mittelmodul 14 zur Anwendung kommen.

Mittels des Handschuhs, an dem die mindestens eine Fingerschiene der Handorthese befestigt oder in dem diese integriert ist, ist sowohl eine gute Kraftübertragung von der Handorthese auf den oder die erkrankten Finger der Hand wie auch ein guter Tragekomfort erreicht. Entsprechend ist der konstruktive Aufbau der erfindungsgemäßen Handorthese sehr einfach und damit kostengünstig. Über die geeignete Anbringung von Befestigungslaschen und -taschen lässt sich die mindestens eine Fingerschiene relativ zu dem zugeordneten, also zu dem zu therapierenden, Finger sicher positionieren.

Der Handschuh ist in dem erläuterten Ausführungsbeispiel als ein bis auf die Fingerkuppen vollständiger Handschuh ausgebildet. Grundsätzlich ist es jedoch denkbar, dass lediglich der oder die zu therapierenden Finger behandschuht sind oder dass die nicht betroffenen Finger lediglich teilweise durch den Handschuh umschlossen sind. Dies kann sowohl über die gesamte Fingerlänge wie auch über lediglich eine Teillänge des Fingers der Fall sein.

Auch ist es möglich, den Handschuh als einen Universalhandschuh auszubilden, der Befestigungselemente, beispielsweise Befestigungslaschen und -taschen oder dergleichen, zur Aufnahme von einer Fingerschiene oder von mehreren Fingerschienen für eine Mehrzahl von Fingern vorsieht. Der Handschuh des Ausführungsbeispiels weist die Befestigungselemente für die Fingerschiene auf dessen Handaußenseite auf. In anderen Anwendungsfällen könnten die Befestigungselemente jedoch auch auf der Handinnenseite des Handschuhs oder auf der Handinnenseite und der Handaußenseite des Handschuhs angeordnet sein.

Durch die erfindungsgemäße Handorthese ist die auf den zu therapierenden Finger ausgeübte Zug- oder Druckbelastung gleichmäßiger auf diesen verteilt. Ferner ist eine sichere Positionierung der Fingerschiene erleichtert.

Anstelle von Nietverbindungen 6 zwischen der Fingerplatte 4 und dem ersten und dem zweiten Federpaket 8, 10 sind auch andere dem Fachmann bekannte und geeignete Verbindungstechniken möglich.

Die Fingerschiene der Handorthese muss sich nicht zwingend bis zur Fingerbeere des betroffenen Fingers erstrecken. Jedoch ist eine Erstreckung bis zum Endglied des zugeordneten Fingers vorteilhaft, da auf diese Weise die Belastung des Fingers durch die Handorthese noch besser verteilt ist und eine bessere Hebelwirkung erzielt wird.

Die bombierte Ausführung der Blattfedern 8.1, 10.1 ist exemplarisch für das Ausführungsbeispiel nach Fig. 5 dargelegt. Erfindungsgemäß kann die bombierte Ausbildung der Blattfedern 8.1, 10.1 auch für die Fingerschiene nach den Fig. 1 bis 4 sowie für jede andere Fingerschiene Verwendung finden. Die Bombierung ist insofern auch nicht beschränkt auf die Verwendung des Mittelmoduls 14. Die Bombierung kann insbesondere bei der Vorsehung der Fingerplatte 4 verwendet werden und/oder zusammen mit einer Nietverbindung 6 für die Federpakete 8, 10.

Die mindestens eine Feder der Fingerschiene muss nicht den Aufbau des Ausführungsbeispiels aufweisen. Beispielsweise ist es denkbar, dass lediglich eine einzige Feder oder ein aus mehreren geschichteten Blattfedern bestehendes einziges Federpaket verwendet wird. Die Fingerschiene könnte also auch aus einer einzigen Feder oder einem einzigen Federpaket bestehen. Analog zu der Fingerschiene des Ausführungsbeispiels könnte sich diese Feder oder dieses Federpaket in einer Gebrauchslage der Handorthese von der Mittelhand, über das Grundgelenk, das Grundglied und das Mittelgelenk bis zumindest zum Mittelglied, vorzugsweise bis zu dem Endglied, des zugeordneten Fingers erstrecken.

In einer Anfangsphase der Therapie werden beispielsweise sechs bis 25 Federn in einem Paket gebraucht, um die nötigen Kräfte bereitzustellen. In einer späteren Phase der Therapie könnte dann die Federkraft beispielsweise einer einzelnen Feder ausreichend sein, um eine Restbegradigung des zu therapierenden Fingers zu erreichen oder eine beispielsweise nach einer Operation erreichte Begradigung zu erhalten.

Alternativ zu dem anhand der Fig. 1 bis 10 erläuterten Ausführungsbeispiel kann die einzige Feder oder das einzige Federpaket im Bereich des Grundgliedes des zugeordneten Fingers derart zweifach geknickt ausgebildet sein, dass die Feder oder das Federpaket einen im Wesentlichen starren Mittelbereich aufweist, von dem aus sich ein erster federnder Endbereich in Richtung der Mittelhand und sich ein zweiter federnder Endbereich in Richtung des Mittelgliedes des zugeordneten Fingers erstreckt.

Bei einem Federpaket wären demnach die einzelnen Blattfedern des Federpakets im einfachsten Fall lediglich ineinandergelegt. Durch die spezielle Ausbildung jeder Blattfeder mit den beiden Knickstellen und den auf diese Weise gebildeten Mittelbereich würden die einzelnen Blattfedern, die in den Therapiehandschuh als Federpaket eingeschoben worden sind, ihre Position in dem Federpaket auch bei dem Gebrauch der Handorthese automatisch beibehalten. Denkbar wäre jedoch auch, dass die einzelnen Blattfedern des Federpakets durch Befestigungsmittel, wie Niete oder dergleichen, zueinander fixiert sind. Beispielsweise könnte an jedem Ende des durch die Knickstellen gebildeten Mittelbereichs eine Nietverbindung vorgesehen sein.

Dieser Mittelbereich würde dann die Funktion der Fingerplatte 4 des Ausführungsbeispiels übernehmen. Um die Steifigkeit des so gebildeten Mittelbereichs zu erhöhen, wäre es denkbar, an den Mittelbereich der Feder eine Versteifungsplatte anzuordnen. Diese Versteifungsplatte könnte beispielsweise in Analogie zu dem Ausführungsbeispiel mit zwei Nietverbindungen an der Feder oder dem Federpaket befestigt sein.

Eine andere alternative Ausführungsform sieht vor, dass die Fingerschiene an einer Grundplatte befestigt ist, wobei die Grundplatte durch den Handschuh im Bereich der Mittelhand fixierbar ist und sich die Fingerschiene, insbesondere die Feder, in der Gebrauchslage der Handorthese von der Grundplatte, über das Grundgelenk, das Grundglied und das Mittelgelenk bis zumindest zum Mittelglied, insbesondere bis zum Endglied, des zugeordneten Fingers erstreckt. Bei dieser Ausführungsform wäre somit lediglich ein freies Ende der Feder oder des Federpaketes und damit nur ein federndes Ende ausgebildet.

Im Gegensatz zu bekannten Handorthesen kann die erfindungsgemäße Handorthese besser für die Verwendung in einem späteren Stadium der Therapie, also bei bereits weiter gestrecktem Finger, angepasst werden. Die erfindungsgemäße Handorthese weist ein geringeres Gewicht auf, schränkt die Bewegungsfreiheit der betroffenen Hand und des betroffenen Fingers weniger ein und bietet somit einen höheren Tragekomfort.

Neben den für die Fingerplatte und die Blattfedern genannten Materialien sind auch andere dem Fachmann bekannte und geeignete Materialien denkbar. Auch ist eine Kombination von voneinander verschiedenen Materialien für die Fingerplatte, die Blattfedern, die Federpakete und andere Bauteile der erfindungsgemäßen Handorthese möglich.

Optional kann nach der Erfindung vorgesehen sein, dass an der Fingerschiene 2 auf einer in der Gebrauchslage dem Finger und/oder dem Handrücken zugewandten Seite wenigstens abschnittsweise ein Silikonpolster vorgesehen ist. Das Silikonpolster kann eine weiche Anlagefläche bereitstellen und zur Verbesserung des Tragekomforts beitragen.

### Bezugszeichenliste

- 2: Fingerschiene
- 4: Fingerplatte
- 6: Nietverbindung
- 8: Erste Feder, als erstes Federpaket aus mehreren Blattfedern ausgebildet
- 8.1: Blattfeder des ersten Federpakets
- 8.2: Ausnehmung
- 10: Zweite Feder, als zweites Federpaket aus mehreren Blattfedern ausgebildet
- 10.1: Blattfeder des zweiten Federpakets
- 10.2: Ausnehmung
- 12: Handschuh
- 12.1: Befestigungstasche, als Einstecktasche ausgebildet
- 12.2: Befestigungstasche, als Einstecktasche ausgebildet
- 12.3: Befestigungslasche, als Klettverschluss ausgebildet
- 12.4: Halteband
- 12.5: flexibles Band
- 12.6: Klettband
- 12.7: Spanngurt mit eingelegtem Federelement
- 14: Mittelmodul
- 14.1.: Stift
- 14.2: Andrückplatte
- 14.3: Schraube

## Patentansprüche

1. Handorthese mit einem Handschuh und mindestens einer Fingerschiene, wobei die Fingerschiene durch den Handschuh an einer Hand fixierbar ist, **dadurch gekennzeichnet, dass** die Fingerschiene (2) mindestens eine Feder (8, 10) aufweist.

2. Handorthese nach Anspruch 1, **dadurch gekennzeichnet, dass** die Feder (8, 10) als eine Blattfeder, insbesondere als ein Federpaket (8, 10) aus mindestens zwei geschichteten Blattfedern (8.1, 10.1), ausgebildet ist, wobei die Blattfeder (8, 10) bevorzugt aus einem flexiblen, elastisch verformbaren Stahl und insbesondere aus einem Federstahl und/oder Bandmaßstahl hergestellt ist.

3. Handorthese nach Anspruch 2, **dadurch gekennzeichnet, dass** das Federpaket (8, 10) derart ausgebildet ist, dass der Zug oder Druck auf den mit der Fingerschiene (2) gestreckten Finger vom proximalen zum distalen Ende des Fingers abnimmt.

4. Handorthese nach einem der Ansprüche 1 bis 3, **dadurch gekennzeichnet, dass** sich die Fingerschiene (2), insbesondere die Feder, in einer Gebrauchslage der Handorthese von der Mittelhand, über das Grundgelenk, das Grundglied und das Mittelgelenk bis zumindest zum Mittelglied, insbesondere bis zum Endglied, des zugeordneten Fingers erstreckt.

5. Handorthese nach Anspruch 4, **dadurch gekennzeichnet, dass** die Feder im Bereich des Grundgliedes des zugeordneten Fingers derart zweifach geknickt ausgebildet ist, dass die Feder einen im Wesentlichen starren Mittelbereich aufweist, von dem aus sich ein erster federnder Endbereich in Richtung der Mittelhand und ein zweiter federnder Endbereich in Richtung des Mittelgliedes des zugeordneten Fingers erstreckt.

6. Handorthese nach Anspruch 5, **dadurch gekennzeichnet, dass** an den Mittelbereich der Feder eine Versteifungsplatte angeordnet ist.

7. Handorthese nach Anspruch 4, **dadurch gekennzeichnet, dass** die Fingerschiene (2) im Bereich des Grundgliedes des zugeordneten Fingers eine Fingerplatte (4) aufweist, wobei an der Fingerplatte (4) mindestens eine erste und mindestens eine zweite Feder (8, 10) angeordnet sind, von denen sich die erste Feder (8) in Richtung der Mittelhand und die zweite Feder (10) in Richtung des Mittelgliedes des zugeordneten Fingers erstreckt.

8. Handorthese nach Anspruch 7, **dadurch gekennzeichnet, dass** die erste Feder (8) als ein Federpaket (8) mit im Wesentlichen gleichlangen Federn (8.1) und/oder die zweite Feder (10) als ein Federpaket (10) mit mindestens einer Innenfeder (10.1) und zwei die Innenfeder (10.1) in der Länge überragenden, insbesondere gleichlangen, Außenfedern (10.1) ausgebildet ist.

9. Handorthese nach einem der Ansprüche 7 oder 8, **dadurch gekennzeichnet, dass** die erste Feder (8) im Wesentlichen zu einem Drittel im Bereich des Grundgliedes des zugeordneten Fingers und zu zwei Drittel im Bereich der Mittelhand angeordnet ist.

10. Handorthese nach einem der Ansprüche 7 bis 9, **dadurch gekennzeichnet, dass** die Fingerplatte (4) als Teil eines Mittelmoduls (14) ausgebildet ist und dass die Federn (8, 10) an dem Mittelmodul (14) zwischen der Fingerplatte (4) und einer Andrückplatte (14.3) des Mittelmoduls (14) festgelegt sind, wobei die Fingerplatte (4) und die Andrückplatte (14.3) lösbar miteinander verbunden sind.

11. Handorthese nach einem der Ansprüche 1 bis 10, **dadurch gekennzeichnet, dass** die Federn (8, 10) bombiert ausgebildet sind mit einer Wölbung quer zu einer Längsrichtung der Fingerschiene (2), wobei bevorzugt die Wölbung in die Längsrichtung der Fingerschiene (2) gleich ausgeformt ist.

12. Handorthese nach Anspruch 11, **dadurch gekennzeichnet, dass** die Fingerschiene (2) in der Gebrauchslage der Handorthese mit einer hohlen Seite der Federn (8, 10) von oben an dem zugeordneten Finger angelegt ist.

13. Handorthese nach einem der Ansprüche 1 bis 12, **dadurch gekennzeichnet, dass** die Fingerschiene an einer Grundplatte befestigt ist, wobei die Grundplatte durch den Handschuh im Bereich der Mittelhand fixierbar ist und sich die Fingerschiene, insbesondere die Feder, in der Gebrauchslage der Handorthese von der Grundplatte, über das Grundgelenk, das Grundglied und das Mittelgelenk bis zumindest zum Mittelglied, insbesondere bis zum Endglied, des zugeordneten Fingers erstreckt.
